**Europäisches Patentamt**

**European Patent Office**

**Office européen des brevets**

(19)

(11) Veröffentlichungsnummer : **0 308 359 B1**

(12)

# EUROPÄISCHE PATENTSCHRIFT

(45) Veröffentlichungstag der Patentschrift :
**12.08.92 Patentblatt 92/33**

(51) Int. Cl.$^5$ : **A61K 6/08**

(21) Anmeldenummer : **88710026.1**

(22) Anmeldetag : **07.09.88**

(54) **Hitzehärtbare Dentalmassen.**

(30) Priorität : **15.09.87 DE 3730921**

(43) Veröffentlichungstag der Anmeldung :
**22.03.89 Patentblatt 89/12**

(45) Bekanntmachung des Hinweises auf die Patenterteilung :
**12.08.92 Patentblatt 92/33**

(84) Benannte Vertragsstaaten :
**AT BE CH DE ES FR GB GR IT LI LU NL SE**

(56) Entgegenhaltungen :
**EP-A- 0 059 649**
**EP-A- 0 127 758**
**EP-A- 0 150 952**
**DE-A- 2 437 698**

(73) Patentinhaber : **Degussa Aktiengesellschaft**
**Weissfrauenstrasse 9**
**W-6000 Frankfurt am Main 1 (DE)**

(72) Erfinder : **Janda, Ralf, Dr.**
**Seulberger Str. 19**
**W-6380 Bad Homburg (DE)**
Erfinder : **Dankowski, Manfred, Dr.**
**Finkenweg 24**
**W-8752 Mömbris-Königshofen (DE)**

EP 0 308 359 B1

## Beschreibung

Die Erfindung betrifft hitzehärtbare Dentalmassen, die neben anderen in Dentalmassen üblicherweise enthaltenen Bestandteilen mindestens einen monomeren Ester der Methacrylsäure und einen peroxidischen Polymerisationskatalysator umfassen.

Derartige Dentalmassen dienen zur Herstellung von künstlichen Zähnen, Zahnteilen oder Prothesenplatten. Sie enthalten außer mindestens einem monomeren und polymerisierbaren Ester der Methacrylsäure und einem in der Wärme die Polymerisation auslösenden peroxidischen Katalysator unter anderem Füllstoffe, Pigmente, Silankupplungsmittel, Stabilisatoren und vorgebildete Polymerisate.

Die Füllstoffe sind häufig mikrofeine anorganische Materialien mit einer Teilchengröße zwischen etwa 0,01 und 5 μm, insbesondere Siliziumdioxid, aber auch Mischungen von Siliziumdioxid mit Aluminiumoxid, Boroxid, Titandioxid oder Zirkonoxid. Sie können, zumindest teilweise, auch so in die Dentalmasse eingebracht werden, daß man ein Polymerisat herstellt, das im wesentlichen aus Estern der Methacrylsäure besteht, unvernetzt oder vernetzt ist und die, gegebenenfalls oberflächenbehandelten, Füllstoffe enthält. Wird das Polymerisat als Perlpolymerisat hergestellt, kann es der Dentalmasse in dieser Form zugesetzt werden, wird es durch Substanzpolymerisation in kompakter Form hergestellt, so wird es anschließend erst zu einem sogenannten Splitterpolymerisat vermahlen. Andere Füllstoffe sind beispielsweise gemahlene Gläser oder Quarz mit mittleren Teilchengrößen zwischen etwa 1 und 10 μm.

Die Pigmente dienen, in geringer Menge zugesetzt, dazu, die Farbe der Dentalmasse mit den verschiedenen Schattierungen natürlicher Zähne oder im Falle von Prothesenplatten mit dem Zahnfleisch in Übereinstimmung zu bringen. Geeignete Pigmente sind beispielsweise Eisenoxidschwarz, Cadmiumgelb und -orange, Zinkoxid und Titandioxid.

Silankupplungsmittel sind Materialien, die zumindest eine polymerisierbare Doppelbindung zur Reaktion mit den monomeren Estern der Methacrylsäure aufweisen. Ihre Funktion ist es, die Haftung zwischen dem Polymerisat und den Füllstoffen zu verbessern. Normalerweise werden die Füllstoffe mit diesen Silankupplungsmitteln vorbehandelt und in dieser Form eingesetzt. Geeignete Silankupplungsmittel sind beispielsweise Vinyltrichlorsilan, Tris-(2-methoxyethoxy)-vinylsilan, Tris-(acetoxy)-vinylsilan und 3-Methacryloxypropyltrimethoxysilan.

Häufig verwendete vorgebildete Polymerisate sind neben den bereits erwähnten füllstoffhaltigen Perl- und Splitterpolymerisaten Homopolymerisate des Methacrylsäuremethylesters oder, vorzugsweise unvernetzte, Copolymerisate des Methacrylsäuremethylesters mit einem geringeren Anteil an Estern der Methacrylsäure oder Acrylsäure mit 2 bis 12 C-Atomen in der Alkoholkomponente, zweckmäßigerweise in der Form eines Perlpolymerisats. Andere geeignete Polymerisate sind unvernetzte Produkte auf der Basis von Polyurethanen, Polycarbonaten, Polyestern und Polyethern.

Übliche Dentalmassen enthalten ferner mindestens einen monomeren Ester der Methacrylsäure, meist aber ein Gemisch aus mehreren solchen Estern. Geeignete monofunktionelle Ester der Methacrylsäure sind beispielsweise Methylmethacrylat, Ethylmethacrylat, Isopropylmethacrylat, n-Hexylmethacrylat und 2-Hydroxy-ethylmethacrylat. Neuerdings werden aber häufig auch mehrfunktionelle Ester der Methacrylsäure eingesetzt, wie Ethylenglykoldimethacrylat, Butandiol-1,4-dimethacrylat, Triethylenglykoldimethacrylat, Dodecandiol-1,12-dimethacrylat, Decandiol-1,10-dimethacrylat, 2,2-Bis-[p($\gamma$-methacryloxy-$\beta$-hydroxypropoxy)-phenyl]-propan, das Diaddukt aus Hydroxyethylmethacrylat und Trimethylhexamethylendiisocyanat, das Diaddukt aus Hydroxyethylmethacrylat und Isophorondiisocyanat, Trimethylolpropantrimethacrylat, Pentaerythrittrimethacrylat, Pentaerythrittetramethacrylat und 2,2-Bis-[p($\beta$-hydroxy-ethoxy)-phenyl]-propandimethacrylat.

Alle diese genannten Bestandteile können auch in den erfindungsgemäßen Dentalmassen enthalten sein, die sich also insoweit von bekannten Dentalmassen nicht unterscheiden.

Bekannte hitzehärtbare Dentalmassen enthalten aber als weiteren wesentlichen Bestandteil ein die Polymerisation auslösendes Peroxid, insbesondere Dibenzoylperoxid (vgl. z.B. EP-A2-127 758). Solche Dibenzoylperoxid enthaltende Dentalmassen sind jedoch nicht im wünschenswerten Maße lagerstabil. Vor allem können auch kurzfristige stärkere Temperaturerhöhungen, z. B. bei Lagerung in einem Lagerhaus, zu einer unerwünschten vorzeitigen Polymerisation führen. In der Regel wird daher für derartige Zubereitungen Kühlschranklagerung vorgeschrieben. Außerdem birgt das Dibenzoylperoxid die Gefahr, daß die fertig ausgehärteten, aus solchen bekannten Dentalmassen hergestellten Dentalformkörper unter Umständen deutlich erkennbare Verfärbungen aufweisen.

Die erfindungsgemäßen Dentalmassen sind nun dadurch gekennzeichnet, daß sie als peroxidischen Polymerisationskatalysator in einer Menge von 0,1 bis 10 Gewichtsprozent, bezogen auf den Gesamtgehalt der Masse an polymerisierbaren Monomeren, mindestens eine Peroxycarbonsäure aus der Gruppe der aliphatischen geradlinigen oder verzweigten Peroxymonocarbonsäuren mit 8 bis 18 C-Atomen, aliphatischen geradlinigen oder verzweigten Monoperoxydicarbonsäuren mit 6 bis 20 C-Atomen, aliphatischen geradlinigen oder verzweigten Diperoxydicarbonsäuren mit 6 bis 20 C-Atomen, aromatischen Peroxymonocarbonsäuren mit 7 bis 12 C-Atomen, aromatischen Monoperoxydicarbonsäuren mit 8 bis 12 C-Atomen und aromatischen Diperoxydicarbonsäuren mit 8 bis 12 C-Atomen enthalten.

Vorzugsweise beträgt der Gehalt der erfindungsgemäßen Dentalmassen an der Peroxycarbonsäure 1 bis 5 Gewichtsprozent, wiederum bezogen auf den Gesamtgehalt der Masse an polymerisierbaren Monomeren.

Beispiele für erfindungsgemäß zu verwendende aliphatische Peroxymonocarbonsäuren sind Peroxycaprylsäure, Peroxycaprinsäure, Peroxylaurinsäure, Peroxymyristinsäure, Peroxypalmitinsäure und Peroxystearinsäure; für aliphatische Monoperoxydicarbonsäuren Monoperoxyadipinsäure, Monoperoxypimelinsäure, Monoperoxykorksäure, Monoperoxyazelainsäure, Monoperoxysebacinsäure, Monoperoxynonandicarbonsäure, Monoperoxydecandicarbonsäure, Monoperoxydodecandicarbonsäure und Monoperoxyhexadecylbernsteinsäure; für aliphatische Diperoxydicarbonsäuren Diperoxyadipinsäure, Diperoxypimelinsäure, Diperoxykorksäure, Diperoxyazelainsäure, Diperoxysebacinsäure, Diperoxynonandicarbonsäure, Diperoxydecandicarbonsäure, Diperoxydodecandicarbonsäure und Hexadecyldiperoxybernsteinsäure; für aromatische Peroxymonocarbonsäuren Peroxybenzoesäure, die Peroxytoluylsäuren und die Peroxynaphthalinmonocarbonsäuren; für aromatische Monoperoxydicarbonsäuren die Monoperoxyphthalsäuren und die Monoperoxynaphthalsäure; für aromatische Diperoxydicarbonsäuren die Diperoxyphthalsäuren und die Diperoxynaphthalsäure.

Im übrigen werden die erfindungsgemäßen Dentalmassen genauso verarbeitet wie herkömmliche. Sie werden in pastöser Form eingesetzt und unter Formgebung durch radikalische Polymerisation zu den gewünschten Dentalformkörpern ausgehärtet. Die zweckmäßigen Härtetemperaturen liegen im Bereich von etwa 90 bis 180 °C, vorzugsweise im Bereich von etwa 90 bis 160 °C, insbesondere im Bereich von etwa 130 bis 160 °C.

Die vollständig ausgehärteten erfindungsgemäßen Dentalmassen weisen, insbesondere dann, wenn die Härtung bei einer Temperatur von 130 °C oder darüber vorgenommen wurde, mechanische Eigenschaften, wie Vickershärte, Biegefestigkeit, Biegemodul und Druckfestigkeit, auf, die denen ausgehärteter herkömmlicher Dentalmassen entsprechen. Aber die fertigen Dentalformkörper sind in vielen Fällen deutlich weniger verfärbt als solche, die mit Dibenzoylperoxid als Polymerisationskatalysator hergestellt wurden.

Ein entscheidender Vorteil der erfindungsgemäßen Dentalmassen liegt jedoch darin, daß sie auch noch nach längerer Lagerung bei Raumtemperatur oder bei Temperaturen bis etwa 40 °C völlig stabil sind und problemlos zu Dentalformkörpern verarbeitet werden können, welche die zu fordernden Eigenschaften aufweisen.

Die Erfindung wird durch die nachfolgenden Beispiele näher erläutert:

Beispiel 1:

Für die Prüfungen wurde eine pastenförmige Dentalmasse folgender Zusammensetzung (Angabe in Gewichtsprozent) eingesetzt:

25 Monomermischung
25 hochdisperses Siliziumdioxid, silanisiert
49 Splitterpolymerisat
1 Polymerisationskatalysator.

Die Monomermischung bestand aus (Angaben in Gewichtsprozent):

60 Methacrylsäure-8,10,10-trimethyl-4,13-dioxo-3,14-dioxa-5,12-diaza-hexadecan-diyl-ester

40 Dodecandiol-1,12-dimethacrylat.

Das hochdisperse Siliziumdioxid mit einer Teilchengröße von 0,01 bis 0,04 μm war in an sich bekannter Weise durch Behandlung mit 3-Methacryloyloxypropyltrimethoxysilan silanisiert.

Das Splitterpolymerisat wurde hergestellt aus (Angabe in Gewichtsprozent):

60 hochdisperses Siliziumdioxid, silanisiert (wie erwähnt)
39 Dodecandiol-1,12-dimethacrylat
1 Peroxycaprinsäure.

Diese Bestandteile wurden zu einem Teig verknetet, der Teig wurde zu Platten ausgewalzt, und die Platten wurden in einem Umlufttrockenschrank bei 130 °C ausgehärtet. Das ausgehärtete Material wurde dann auf eine Teilchengröße von etwa 100 μm vermahlen.

Die obige pastenförmige Dentalmasse wurde unter Verwendung verschiedener Peroxycarbonsäuren und (zum Vergleich) von Dibenzoylperoxid in einem Umlufttrockenschrank bei 130 °C und 160 °C 30 Minuten lang ausgehärtet, und es wurde die Vickershärte HV 5 gemessen. Die Ergebnisse sind in Tabelle 1 zusammenge-

stellt:

Tabelle 1

| Polymerisations-katalysator | $HV5(N/mm^2)$ nach Härtung bei | |
|---|---|---|
| | $130\ °C$ | $160°C$ |
| Peroxycaprinsäure | 293 | 311 |
| Peroxystearinsäure | 286 | 288 |
| Diperoxyadipinsäure | 246 | 319 |
| Diperoxyazelainsäure | 285 | 308 |
| Diperoxydodecandisäure | 284 | 284 |
| Hexadecyldiperoxy-bernsteinsäure | 225 | 276 |
| Dibenzoylperoxid | 299 | 299 |

Beispiel 2:

Eingesetzt wurde eine pastenförmige Dentalmasse folgender Zusammensetzung (Angaben in Gewichtsprozent):

10  2,2-Bis-[4-(3'-methacryloyloxy-2'-hydroxy-propoxy)-phenyl]-propan
7  Methacrylsäure-8,10,10-trimethyl-4,13-dioxo-3,14-dioxa-5,12-diaza-hexadecan-diyl-ester
7  Triethylenglykoldimethacrylat
75  Aluminiumsilikatglaspulver einer mittleren Teilchengröße von 6 µm
1  Polymerisationskatalysator.

Diese pastenförmige Dentalmasse wurde unter Verwendung verschiedener Peroxycarbonsäuren und (zum Vergleich) von Dibenzoylperoxid in einem Umlufttrockenschrank bei 160 °C 30 Minuten lang ausgehärtet, und es wurde die Vickershärte HV5 gemessen. Die Ergebnisse sind in Tabelle 2 zusammengestellt:

Tabelle 2

| Polymerisations-katalysator | $HV5(N/mm^2)$ |
|---|---|
| Peroxycaprinsäure | 750 |
| Diperoxyazelainsäure | 680 |
| Diperoxydodecandisäure | 240 |
| Dibenzoylperoxid | 780 |

Beispiel 3:

An der pastenförmigen Dentalmasse gemäß Beispiel 1 wurde unter Verwendung verschiedener Peroxycarbonsäuren und (zum Vergleich) von Dibenzoylperoxid die Lagerstabilität bei 37 °C und 60 °C gemessen. Die Ergebnisse sind in Tabelle 3 zusammengestellt:

Tabelle 3

| Polymerisations-katalysator | Lagerstabilität bei | |
|---|---|---|
| | 37 °C | 60 °C |
| Peroxycaprinsäure | > 5 Monate | 3 Tage |
| Peroxystearinsäure | " | 1 Tag |
| Diperoxyadipinsäure | " | 3 Tage |
| Diperoxyazelainsäure | " | 2 Tage |
| Diperoxydodecandisäure | " | 3 Tage |
| Hexadecyldiperoxy-bernsteinsäure | " | 14 Tage |
| Dibenzoylperoxid | 16 Tage | 3 Stunden |

Beispiel 4:

Unter Verwendung verschiedener Peroxycarbonsäuren und (zum Vergleich) von Dibenzoylperoxid wurden pastenförmige Dentalmassen gemäß Beispiel 1 hergestellt und 9 Monate lang bei Raumtemperatur gelagert. Dann wurden sie, wie im Beispiel 1 angegeben, ausgehärtet, und es wurde die Vickershärte HV5 gemessen. Die Ergebnisse sind in Tabelle 4 zusammengestellt:

Tabelle 4

| Polymerisations-katalysator | $HV5 (N/mm^2)$ nach Härtung bei | |
|---|---|---|
| | 130 °C | 160 °C |
| Peroxystearinsäure | 295 | 315 |
| Diperoxydodecandisäure | 300 | 330 |
| Hexadecyldiperoxy-bernsteinsäure | 250 | 295 |
| Dibenzoylperoxid | 320 | 330 |

Beispiel 5:

Unter Verwendung verschiedener Peroxycarbonsäuren wurden pastenförmige Dentalmassen gemäß Beispiel 1 hergestellt und 5 Monate lang bei 37 °C gelagert. Dann wurden sie, wie im Beispiel 1 angegeben, ausgehärtet, und es wurde die Vickershärte HV5 gemessen. Die Ergebnisse sind in Tabelle 5 zusammengestellt:

Tabelle 5

| Polymerisation-katalysator | HV5 ($N/mm^2$) nach Härtung bei | |
|---|---|---|
| | 130 °C | 160 °C |
| Peroxycaprinsäure | 290 | 330 |
| Peroxystearinsäure | 310 | 345 |
| Diperoxyadipinsäure | 240 | 300 |
| Diperoxyazelainsäure | 290 | 320 |
| Diperoxydodecandisäure | 310 | 345 |

Beispiel 6:

Unter Verwendung von Peroxycaprinsäure und (zum Vergleich) von Dibenzoylperoxid wurden pastenförmige Dentalmassen gemäß Beispiel 1 hergestellt und zu Prüfkörpern für die Messung der Biegefestigkeit, des Biegemoduls und der Druckfestigkeit verarbeitet. Die Prüfkörper wurden in einem Umlufttrockenschrank bei 130 °C ausgehärtet. Die Biegefestigkeit und der Biegemodul wurden nach DIN 13922 gemessen. Die Druckfestigkeit wurde an 6 mm hohen zylindrischen Prüfkörpern mit einem Durchmesser von 4 mm ermittelt. Die Ergebnisse sind in Tabelle 6 zusammengestellt:

Tabelle 6

| Polymerisations-katalysator | Biege-festigkeit ($N/mm^2$) | Biege-modul ($N/mm^2$) | Druck-festigkeit ($N/mm^2$) |
|---|---|---|---|
| Peroxycaprinsäure | 55 | 3900 | 300 |
| Dibenzoylperoxid | 55 | 3400 | 305 |

Beispiel 7:

Unter Verwendung von Peroxycaprinsäure und (zum Vergleich) von Dibenzoylperoxid wurden pastenförmige Dentalmassen gemäß Beispiel 2 hergestellt und zu Prüfkörpern für die Messung der Biegefestigkeit, des Biegemoduls und der Druckfestigkeit verarbeitet. Es wurde verfahren wie im Beispiel 6, aber die Aushärtung der Prüfkörper erfolgte bei 160 °C. Die Ergebnisse sind in Tabelle 7 zusammengestellt:

Tabelle 7

| Polymerisations-katalysator | Biege-festigkeit $(N/mm^2)$ | Biege-modul $(N/mm^2)$ | Druck-festigkeit $(N/mm^2)$ |
|---|---|---|---|
| Peroxycaprinsäure | 125 | 10000 | 280 |
| Dibenzoylperoxid | 118 | 10000 | 280 |

Beispiel 8:

Die verwendete Dentalmasse wurde hergestellt aus einer Flüssigkeit und einer Pulvermischung. Die Flüssigkeit war reines monomeres Methylmethacrylat und die Pulvermischung bestand aus (Angaben in Gewichtsprozent):

80      Polymethylmethacrylat vom Molekulargewicht ca. 450000 (M 449, Röhm GmbH)
19,5    Polymethylmethacrylat vom Molekulargewicht ca. 450000, weichmacherhaltig (MW 422, Röhm GmbH)
0,5     Polymerisationskatalysator.

Pulvermischung und monomeres Methylmethacrylat wurden im Verhältnis 3 : 1 (W/W) angerührt. Nach 10 Minuten wurde der angequollene Teig in Formen gepreßt und in einem Wasserbad bei 90 °C und 4 bar Druck eine Stunde lang polymerisiert. Als Polymerisationskatalysatoren dienten Diperoxyazelainsäure und (zum Vergleich) Dibenzoylperoxid. Gemessen wurden die Kugeldruckhärte nach DIN 53456 und die Biegespannung und die Schlagzähigkeit nach DIN 53435. Die Ergebnisse sind in Tabelle 8 zusammengestellt:

Tabelle 8

| Polymerisations-katalysator | Kugeldruck-härte $(N/mm^2)$ | Biege-spannung $(N/mm^2)$ | Schlag-zähigkeit $(kJ/m^2)$ |
|---|---|---|---|
| Diperoxyazelainsäure | 150 | 99 | 5,1 |
| Dibenzoylperoxid | 147 | 92 | 4,9 |

Beispiel 9:

Unter Verwendung verschiedener Peroxycarbonsäuren und (zum Vergleich) von Dibenzoylperoxid wurden pastenförmige Dentalmassen gemäß Beispiel 1 hergestellt und zu Prüfkörpern eines Durchmessers von 20 mm und einer Dicke von 0,5 mm verarbeitet. Die Aushärtung der Prüfkörper erfolgte bei 160 °C.

Zur Bestimmung der Farbstabilität der ausgehärteten Prüfkörper wurden die Bestrahlungseinheit und die Bestrahlungsanordnung nach DIN 13931 verwendet. Die Prüfkörper wurden vor und nach der Bestrahlung mit einem Spektralphotometer vermessen, und es wurden die CieLab-Werte nach DIN 5033, Teil 3, berechnet. Aus der Differenz dieser Werte wurde $\Delta E$ als Maß für die Gesamtfarbänderung errechnet. Die $\Delta E$-Werte sind in Tabelle 9 zusammengestellt:

Tabelle 9

| Polymerisations-katalysator | $\Delta$ E-Wert |
|---|---|
| Peroxycaprinsäure | 0,2 |
| Peroxystearinsäure | 0,4 |
| Diperoxyadipinsäure | 0,8 |
| Diperoxyazelainsäure | 0,5 |
| Diperoxydodecandisäure | 0,5 |
| Hexadecyldiperoxy-bernsteinsäure | 1,0 |
| Dibenzoylperoxid | 3,5 |

Beispiel 10:

Unter Verwendung verschiedener Peroxycarbonsäuren und (zum Vergleich) von Dibenzoylperoxid wurden pastenförmige Dentalmassen gemäß Beispiel 2 hergestellt und, wie in Beispiel 9 angegeben, zu Prüfkörpern für die Bestimmung der Farbstabilität verarbeitet. Die $\Delta$E-Werte sind in Tabelle 10 zusammengestellt:

Tabelle 10

| Polymerisations-katalysator | $\Delta$ E-Wert |
|---|---|
| Peroxycaprinsäure | 0,5 |
| Diperoxyazelainsäure | 3,0 |
| Diperoxydodecandisäure | 2,0 |
| Dibenzoylperoxid | 6,0 |

**Patentansprüche**

1. Hitzehärtbare Dentalmassen, die neben anderen in Dentalmassen üblicherweise enthaltenen Bestandteilen mindestens einen monomeren Ester der Methacrylsäure und einen peroxidischen Polymerisationskatalysator umfassen, dadurch gekennzeichnet, daß sie als peroxidischen Polymerisationskatalysator in einer Menge von 0,1 bis 10 Gewichtsprozent, bezogen auf den Gesamtgehalt der Masse an polymerisierbaren Monomeren, mindestens eine Peroxycarbonsäure aus der Gruppe der aliphatischen geradlinigen oder verzweigten Peroxymonocarbonsäuren mit 8 bis 18 C-Atomen, aliphatischen geradlinigen oder verzweigten Monoperoxydicarbonsäuren mit 6 bis 20 C-Atomen, aliphatischen geradlinigen oder verzweigten Diperoxydicarbonsäuren mit 6 bis 20 C-Atomen, aromatischen Peroxymonocarbonsäuren mit 7 bis 12 C-Atomen, aromatischen Monoperoxydicarbonsäuren mit 8 bis 12 C-Atomen und aromatischen Diperoxydicarbonsäuren mit 8 bis 12 C-Atomen enthalten.

2. Hitzehärtbare Dentalmassen nach Anspruch 1, dadurch gekennzeichnet, daß sie die Peroxycarbonsäure in einer Menge von 1 bis 5 Gewichtsprozent enthalten.

## Claims

1. Thermocurable dental compositions which contain at least one monomeric ester of methacrylic acid and a peroxidic polymerization catalyst in addition to other components usually present in dental compositions, characterised in that they contain, as peroxidic polymerization catalyst, 0.1 to 10 per cent by weight, relative to the total content of polymerizable monomers in the composition, of at least one peroxycarboxylic acid from the series comprising aliphatic straight-chain or branched peroxymonocarboxylic acids having 8 to 18 C atoms, aliphatic straight-chain or branched monoperoxydicarboxylic acids having 6 to 20 C atoms, aliphatic straight-chain or branched diperoxydicarboxylic acids having 6 to 20 C atoms, aromatic peroxymonocarboxylic acids having 7 to 12 C atoms, aromatic monoperoxydicarboxylic acids having 8 to 12 C atoms and aromatic diperoxydicarboxylic acids having 8 to 12 C atoms.

2. Thermocurable dental compositions according to Claim 1, characterised in that they contain 1 to 5 per cent by weight of the peroxycarboxylic acid.

## Revendications

1. Masses dentaires thermodurcissables qui comprennent à côté d'autres constituants contenus habituellement dans les masses dentaires, au moins un ester monomère de l'acide méthacrylique et un catalyseur de polymérisation peroxydique, caractérisées en ce qu'elles renferment comme catalyseur de polymérisation peroxydique en quantité allant de 0,1 à 10 % en poids rapporté à la teneur totale de la masse en monomères polymérisables, au moins un acide peroxycarboxylique choisi dans le groupe des acides peroxymonocarboxyliques aliphatiques linéaires ou ramifiés, ayant de 8 à 18 atomes de carbone, des acides monoperoxydicarboxyliques aliphatiques linéaires ou ramifiés ayant de 6 à 20 atomes de Carbone, des acides peroxymonocarboxyliques aromatiques ayant de 7 à 12 atomes de Carbone, des acides monoperoxydicarboxyliques aromatiques ayant de 8 à 12 atomes de Carbone et des acides diperoxydicarboxyliques aromatiques ayant de 8 à 12 atomes de Carbone.

2. Masses dentaires thermodurcissables selon la revendication 1, caractérisées en ce qu'elles renferment l'acide peroxycarboxylique en quantité allant de 1 à 5 % en poids.